# EUROPEAN PATENT SPECIFICATION

(11) **EP 2 988 669 B1**
(45) Date of publication and mention of the grant of the patent: **24.04.2019**
(21) Application number: 14712388.9
(22) Date of filing: 25.02.2014
(51) Int. Cl.: A61B 5/06, A61B 17/24, A61B 90/00

(54) **NARROWBAND ILLUMINATION OF PARANASAL CAVITIES FOR VISUAL LOCALIZATION OF TARGET TISSUE**
SCHMALBANDIGE BELEUCHTUNG VON NASENNEBENHÖHLEN ZUR VISUELLEN LOKALISIERUNG VON ZIELGEWEBE
ÉCLAIRAGE À BANDE ÉTROITE DE CAVITÉS PARANASALES POUR LA LOCALISATION VISUELLE DE TISSU CIBLE

(30) Priority: 14.03.2013 US 201313827458
(43) Date of publication of application: 02.03.2016
(73) Proprietor: Acclarent, Inc., Irvine, CA 92618 (US)
(72) Inventor: JONES, Jeffrey S., Hillsborough, California 94010 (US); CHAMNESS, Scott O., Menlo Park, California 94025 (US); TRASK, David, Redwood City, California 94061 (US); CHAN, Kin F., Los Gatos, California 95032 (US)
(74) Representative: Tunstall, Christopher Stephen
(86) International application number: PCT/US2014/018358
(87) International publication number: WO 2014/158576

(56) References cited:
- WO-A1-99/64099
- US-A- 4 898 175
- US-A1- 2010 030 031
- US-A1- 2010 036 227
- US-A1- 2012 078 118

## Description

### BACKGROUND

In some instances, it may be desirable to dilate an anatomical passageway in a patient. This may include dilation of ostia of paranasal sinuses (e.g., to treat sinusitis), dilation of the larynx, dilation of the Eustachian tube, dilation of other passageways within the ear, nose, or throat, etc. One method of dilating anatomical passageways includes using a guide wire and catheter to position an inflatable balloon within the anatomical passageway, then inflating the balloon with a fluid (e.g., saline) to dilate the anatomical passageway. For instance, the expandable balloon may be positioned within an ostium at a paranasal sinus and then be inflated, to thereby dilate the ostium by remodeling the bone adjacent to the ostium, without requiring incision of the mucosa or removal of any bone. The dilated ostium may then allow for improved drainage from and ventilation of the affected paranasal sinus. A system that may be used to perform such procedures may be provided in accordance with the teachings of U.S. Pub. No. 2011/0004057, entitled "Systems and Methods for Transnasal Dilation of Passageways in the Ear, Nose or Throat," published January 6, 2011. An example of such a system is the Relieva® Spin Balloon Sinuplasty™ System by Acclarent, Inc. of Menlo Park, California.

A variable direction view endoscope may be used with such a system to provide visualization within the anatomical passageway (e.g., the ear, nose, throat, paranasal sinuses, etc.) to position the balloon at desired locations. A variable direction view endoscope may enable viewing along a variety of transverse viewing angles without having to flex the shaft of the endoscope within the anatomical passageway. Such an endoscope that may be provided in accordance with the teachings of U.S. Pub. No. 2010/0030031, entitled "Swing Prism Endoscope," published February 4, 2010. An example of such an endoscope is the Acclarent Cyclops™ Multi-Angle Endoscope by Acclarent, Inc. of Menlo Park, California.

While a variable direction view endoscope may be used to provide visualization within the anatomical passageway, it may also be desirable to provide additional visual confirmation of the proper positioning of the balloon before inflating the balloon. This may be done using an illuminating guidewire. Such a guidewire may be positioned within the target area and then illuminated, with light projecting from the distal end of the guidewire. This light may illuminate the adjacent tissue (e.g., hypodermis, subdermis, etc.) and thus be visible to the naked eye from outside the patient through transcutaneous illumination. For instance, when the distal end is positioned in the maxillary sinus, the light may be visible through the patient's cheek. Using such external visualization to confirm the position of the guidewire, the balloon may then be advanced distally along the guidewire into position at the dilation site. Such an illuminating guidewire may be provided in accordance with the teachings of U.S. Pub. No. 2012/0078118, entitled "Sinus Illumination Lightwire Device," published March 29, 2012. An example of such an illuminating guidewire is the Relieva Luma Sentry™ Sinus Illumination System by Acclarent, Inc. of Menlo Park, California.

US Patent 4,898,175 discloses an out-body observing apparatus in which an illuminating light fed by an illuminating device is emitted from the tip part of an insertable part of an endoscope inserted into a body cavity, e.g. the stomach, and is radiated onto a part to be observed. This illuminating light having passed through a living body tissue is imaged by an imaging device provided outside the body. This imaging means delivers a picture image signal to a signal processing device. This signal processing device processes the signal and outputs a video signal to a displaying device. This displaying device displays on a picture surface the image of the observed part of the tissue within the living body.

While several instruments and procedures have been made and used for treatment of anatomical passageways in a patient, it is believed that no one prior to the inventors has made or used the invention described in the appended claims.

### BRIEF DESCRIPTION OF THE DRAWINGS

While the specification concludes with claims which particularly point out and distinctly claim the invention, it is believed the present invention will be better understood from the following description of certain examples taken in conjunction with the accompanying drawings, in which like reference numerals identify the same elements and in which:
FIG. 1 depicts a side elevational view of an exemplary dilation catheter system;
FIG. 2 depicts a side elevational view of an exemplary illuminating guidewire suitable for use with the dilation catheter system of FIG. 1;
FIG. 3 depicts a side cross-sectional view of the illuminating guidewire of FIG. 2;
FIG. 4 depicts depicts a perspective view of an exemplary endoscope suitable for use with the dilation catheter system of FIG. 1;
FIG. 5 depicts a side elevational view of the distal end of the endoscope of FIG. 5, showing an exemplary range of viewing angles;
FIG. 6 depicts a schematic view of a patient with an exemplary infrared illuminated guidewire system;
FIG. 7 depicts a front elevational view of exemplary infrared glasses suitable for use with an infrared illuminated guidewire system;
FIG. 8 depicts a schematic view of an embodiment of an infrared illuminated guidewire system according to the present invention; and
FIG. 9 depicts a schematic view of an exemplary alternative illuminated guidewire.

The drawings are not intended to be limiting in any way, and it is contemplated that various embodiments of the invention may be carried out in a variety of other ways, including those not necessarily depicted in the drawings. The accompanying drawings incorporated in and forming a part of the specification illustrate several aspects of the present invention, and together with the description serve to explain the principles of the invention; it being understood, however, that this invention is not limited to the precise arrangements shown.

### DETAILED DESCRIPTION

The following description of certain examples of the technology should not be used to limit its scope. Other examples, features, aspects, embodiments, and advantages of the technology will become apparent to those skilled in the art from the following description, which is by way of illustration, one of the best modes contemplated for carrying out the technology. As will be realized, the technology described herein is capable of other different and obvious aspects, all without departing from the technology. Accordingly, the drawings and descriptions should be regarded as illustrative in nature and not restrictive.

It will be appreciated that the terms "proximal" and "distal" are used herein with reference to a clinician gripping a handpiece assembly. Thus, an end effector is distal with respect to the more proximal handpiece assembly. It will be further appreciated that, for convenience and clarity, spatial terms such as "top" and "bottom" also are used herein with respect to the clinician gripping the handpiece assembly. However, surgical instruments are used in many orientations and positions, and these terms are not intended to be limiting and absolute.

It is further understood that any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

### I. Overview of Exemplary Dilation Catheter System

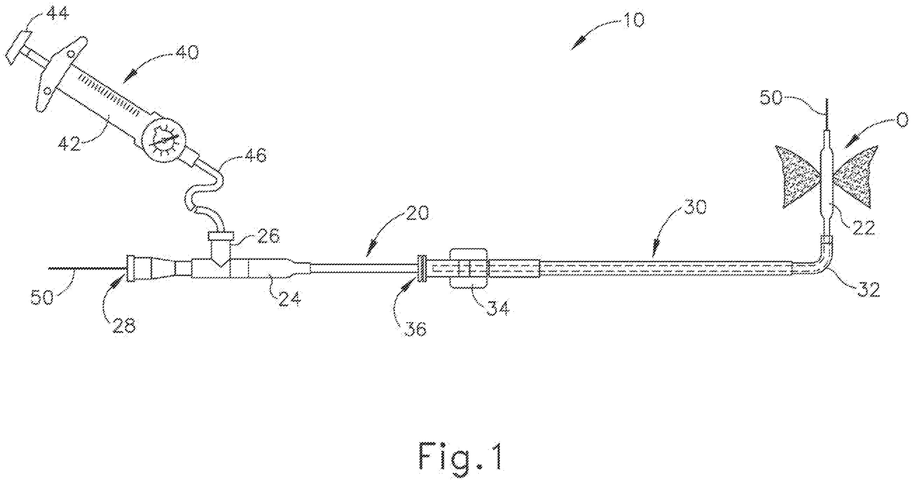

FIG. 1 shows an exemplary dilation catheter system (10) that may be used to dilate the ostium of a paranasal sinus; or to dilate some other anatomical passageway (e.g., within the ear, nose, or throat, etc.). Dilation catheter system (10) of this example comprises a dilation catheter (20), a guide catheter (30), an inflator (40), and a guidewire (50). By way of example only, dilation catheter system (10) may be configured in accordance with at least some of the teachings of U.S. Patent Pub. No. 2011/0004057. In some versions, at least part of dilation catheter system (10) is configured similar to the Relieva® Spin Balloon Sinuplasty™ System by Acclarent, Inc. of Menlo Park, California.

The distal end of dilation catheter (20) includes an inflatable dilator (22). The proximal end of dilation catheter (20) includes a grip (24), which has a lateral port (26) and an open proximal end (28). Dilation catheter (20) includes a first lumen (not shown) that provides fluid communication between lateral port (26) and the interior of dilator (22). Dilator catheter (20) also includes a second lumen (not shown) that extends from open proximal end (28) to an open distal end that is distal to dilator (22). This second lumen is configured to slidably receive guidewire (50). The first and second lumens of dilator catheter (20) are fluidly isolated from each other. Thus, dilator (22) may be selectively inflated and deflated by communicating fluid along the first lumen via lateral port (26) while guidewire (50) is positioned within the second lumen. In some versions, dilator catheter (20) is configured similar to the Relieva Ultirra™ Sinus Balloon Catheter by Acclarent, Inc. of Menlo Park, California. In some other versions, dilator catheter (20) is configured similar to the Relieva Solo Pro™ Sinus Balloon Catheter by Acclarent, Inc. of Menlo Park, California. Other suitable forms that dilator catheter (20) may take will be apparent to those of ordinary skill in the art in view of the teachings herein.

Guide catheter (30) of the present example includes a bent distal end (32) and a grip (34) at its proximal end. Grip (34) has an open proximal end (36). Guide catheter (30) defines a lumen that is configured to slidably receive catheter (20), such that guide catheter (30) may guide dilator (22) out through bent distal end (32). In some versions, guide catheter (30) is configured similar to the Relieva Flex™ Sinus Guide Catheter by Acclarent, Inc. of Menlo Park, California. Other suitable forms that guide catheter (30) may take will be apparent to those of ordinary skill in the art in view of the teachings herein.

Inflator (40) of the present example comprises a barrel (42) that is configured to hold fluid and a plunger (44) that is configured to reciprocate relative to barrel (42) to selectively discharge fluid from (or draw fluid into) barrel (42). Barrel (42) is fluidly coupled with lateral port (26) via a flexible tube (46). Thus, inflator (40) is operable to add fluid to dilator (22) or withdraw fluid from dilator (22) by translating plunger (44) relative to barrel (42). In the present example, the fluid communicated by inflator (40) comprises saline, though it should be understood that any other suitable fluid may be used. In some versions, inflator (40) is configured in accordance with at least some of the teachings of U.S. Pat. App. No. 61/725,523, entitled "Inflator for Dilation of Anatomical Passageway," filed November 13, 2012. Other suitable forms that inflator (40) may take will be apparent to those of ordinary skill in the art in view of the teachings herein.

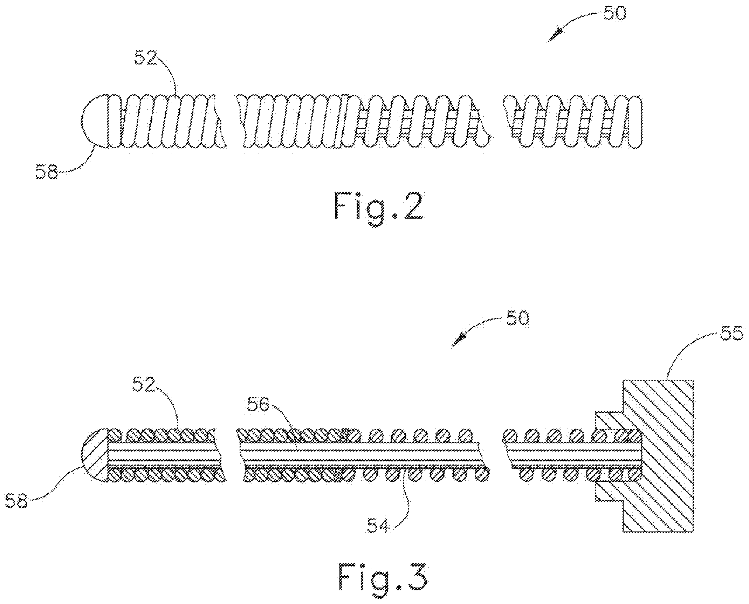

As best seen in FIGS. 2-3, guidewire (50) of the present example comprises a coil (52) positioned about a core wire (54). An illumination fiber (56) extends along the interior of core wire (54) and terminates in an atraumatic lens (58). A connector (55) at the proximnal end of guidewire (50) enables optical coupling between illumination fiber (56) and a light source (not shown). Illumination fiber (56) may comprise one or more optical fibers. Lens (58) is configured to project light when illumination fiber (56) is illuminated by the light source, such that illumination fiber (56) transmits light from the light source to the lens (58). In some versions, the distal end of guidewire (50) is more flexible than the proximal end of guidewire (50). Guidewire (50) has a length enabling the distal end of guidewire (50) to be positioned distal to dilator (22) while the proximal end of guidewire (50) is positioned proximal to grip (24). Guidewire (50) may include indicia along at least part of its length (e.g., the proximal portion) to provide the operator with visual feedback indicating the depth of insertion of guidewire (50) relative to dilation catheter (20). By way of example only, guidewire (50) may be configured in accordance with at least some of the teachings of U.S. Pub. No. 2012/0078118. In some versions, guidewire (50) is configured similar to the Relieva Luma Sentry™ Sinus Illumination System by Acclarent, Inc. of Menlo Park, California. Other suitable forms that guidewire (50) may take will be apparent to those of ordinary skill in the art in view of the teachings herein.

In an exemplary dilation procedure, guide catheter (30) may first be positioned near the targeted anatomical passageway, such as a sinus ostium (O). Dilator (22) and the distal end of guidewire (50) may be positioned within or proximal to bent distal end (32) of guide catheter (30) at this stage. Guide catheter (30) is initially inserted into the nose of the patient and is advanced to a position that is within or near the ostium (O) to be dilated. This positioning of guide catheter (30) may be performed under visualization provided by an endoscope such as endoscope (60) described below. After guide catheter (30) has been positioned, the operator may advance guidewire (50) distally through guide catheter (30) such that a distal portion of the guidewire (50) passes through the sinus ostium (O) and into the sinus cavity. The operator may illuminate illumination fiber (56) and lens (58), which may provide transcutaneous illumination through the patient's face to enable the operator to visually confirm positioning of the distal end of guidewire (50) with relative ease.

With guide catheter (30) and guidewire (50) suitably positioned, dilation catheter (20) is advanced along guidewire (50) and through bent distal end (32) of guide catheter (30), with dilator (22) in a non-dilated state until dilator (22) is positioned within the sinus ostium (O) (or some other targeted anatomical passageway). After dilator (22) has been positioned within the ostium (O), dilator (22) may be inflated, thereby dilating the ostium. To inflate dilator (22), plunger (44) may be actuated to push saline from barrel (42) of inflator (40) through dilation catheter (20) into dilator (22). The transfer of fluid expands dilator (22) to an expanded state to open or dilate the ostium (O), such as by remodeling the bone, etc., forming ostium (O). By way of example only, dilator (22) may be inflated to a volume sized to achieve about 10 to about 12 atmospheres. Dilator (22) may be held at this volume for a few seconds to sufficiently open the ostium (O) (or other targeted anatomical passageway). Dilator (22) may then be returned to a non-expanded state by reversing plunger (44) of inflator (40) to bring the saline back to inflator (40). Dilator (22) may be repeatedly inflated and deflated in different ostia and/or other targeted anatomical passageways. Thereafter, dilation catheter (20), guidewire (50), and guide catheter (30) may be removed from the patient.

### II. Overview of Exemplary Endoscope

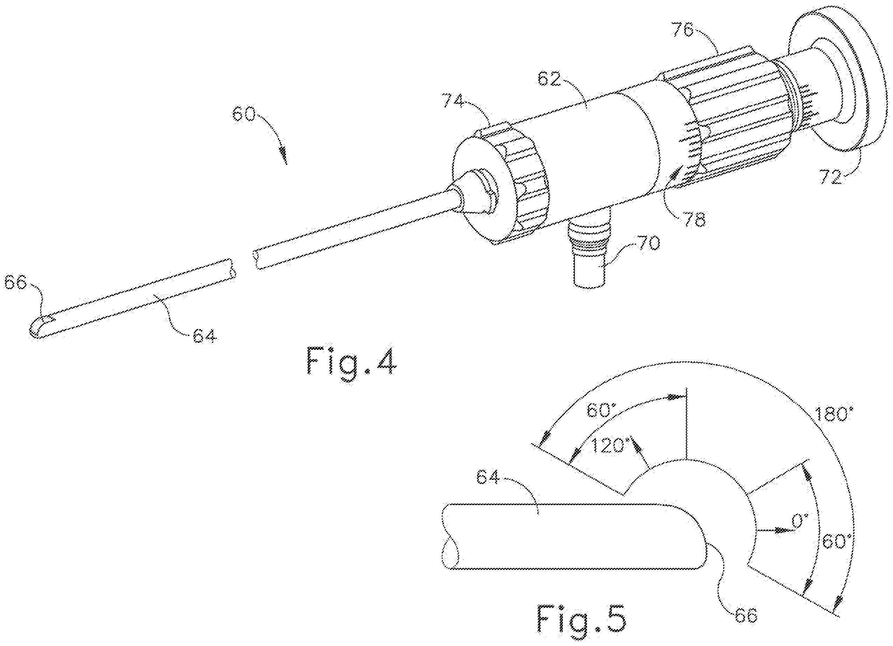

As noted above, an endoscope (60) may be used to provide visualization within an anatomical passageway (e.g., within the nasal cavity, etc.) during a process of using dilation catheter system (10). As shown in FIGS. 4-5, endoscope of the present example comprises a body (62) and a rigid shaft (64) extending distally from body (62). The distal end of shaft (64) includes a curved transparent window (66). A plurality of rod lenses and light transmitting fibers may extend along the length of shaft (64). A lens is positioned at the distal end of the rod lenses and a swing prism is positioned between the lens and window (66). The swing prism is pivotable about an axis that is transverse to the longitudinal axis of shaft (64). The swing prism defines a line of sight that pivots with the swing prism. The line of sight defines a viewing angle relative to the longitudinal axis of shaft (64). This line of sight may pivot from approximately 0 degrees to approximately 120 degrees, from approximately 10 degrees to approximately 90 degrees, or within any other suitable range. The swing prism and window (66) also provide a field of view spanning approximately 60 degrees (with the line of sight centered in the field of view). Thus, the field of view enables a viewing range spanning approximately 180 degrees, approximately 140 degrees, or any other range, based on the pivot range of the swing prism. Of course, all of these values are mere examples.

Body (62) of the present example includes a light post (70), an eyepiece (72), a rotation dial (74), and a pivot dial (76). Light post (70) is in communication with the light transmitting fibers in shaft (64) and is configured to couple with a source of light, to thereby illuminate the site in the patient distal to window (66). Eyepiece (72) is configured to provide visualization of the view captured through window (66) via the optics of endoscope (60). It should be understood that a visualization system (e.g., camera and display screen, etc.) may be coupled with eyepiece (72) to provide visualization of the view captured through window (66) via the optics of endoscope (60). Rotation dial (74) is configured to rotate shaft (64) relative to body (62) about the longitudinal axis of shaft (64). It should be understood that such rotation may be carried out even while the swing prism is pivoted such that the line of sight is non-parallel with the longitudinal axis of shaft (64). Pivot dial (76) is coupled with the swing prism and is thereby operable to pivot the swing prism about the transverse pivot axis. Indicia (78) on body (62) provide visual feedback indicating the viewing angle. Various suitable components and arrangements that may be used to couple rotation dial (74) with the swing prism will be apparent to those of ordinary skill in the art in view of the teachings herein. By way of example only, endoscope (60) may be configured in accordance with at least some of the teachings of U.S. Pub. No. 2010/0030031. In some versions, endoscope (60) is configured similar to the Acclarent Cyclops™ Multi-Angle Endoscope by Acclarent, Inc. of Menlo Park, California. Other suitable forms that endoscope (60) may take will be apparent to those of ordinary skill in the art in view of the teachings herein.

### III. Exemplary Alternative Illuminated Guidewires

In some instances, it may be difficult to see light emitted from a conventional illuminated guidewire (50) that is positioned within a patient's sinus. By way of example, ambient light within the room where the patient is situated, the patient's physiology (e.g., tissue thickness, melanin density, etc.), and/or other factors may make it difficult to see such light with the naked eye from a perspective external to the patient. In addition, a light source that is coupled with a conventional illuminated guidewire (50) may consume a significant amount of power, with the guidewire (50) providing significant losses in electrical-to-optical efficiency. Accordingly, it may be desirable to provide easier external viewing of light emitted by an illuminated guidewire (50) within a patient's sinus in various ambient lighting conditions. It may also be desirable to provide an illuminated guidewire (50) system (i.e., a system formed by a combination of illuminated guidewire (50) and an electrically powered light source) that has a greater electrical-to-optical efficiency than a conventional system. Several exemplary alternative versions of illuminated guidewire (50) systems will be described in greater detail below, while other examples will be apparent to those of ordinary skill in the art in view of the teachings herein.

### A. Exemplary Infrared Illuminated Guidewire

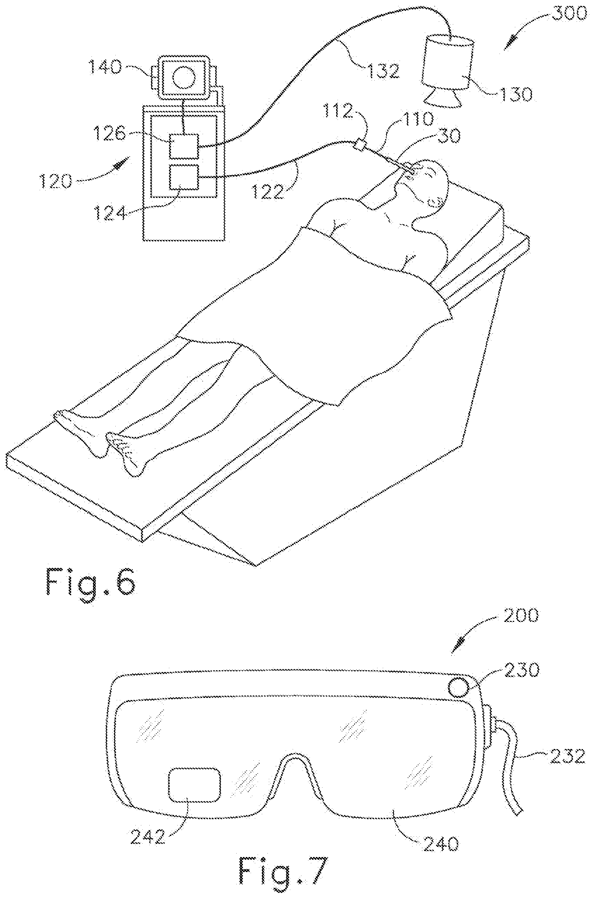

FIG. 6 shows an exemplary illuminated guidewire system (100) that comprises an illuminated guidewire (110), a lighting system (120), a camera (130), and a display (140). While guidewire (50) described above is configured to emit light in the visible spectrum (e.g., wavelengths between approximately 380 nm and approximately 700 nm), guidewire (110) of the present example is configured to emit light in the infrared spectrum (e.g., wavelengths between approximately 700 nm and approximately 1 mm). Guidewire (110) of this example is substantially similar to guidewire (50) described above, though guidewire (110) includes one or more embedded optical fibers that are configured to communicate infrared light. The proximal end of guidewire (110) includes a connector (112) that is configured to couple with an optical cable (122) that extends from lighting system (120). As shown, guidewire (110) is sized for insertion through guide catheter (30) in a patient's nose, just like guidewire (50).

Lighting system (120) of the present example includes a light source (124) and a processor (126). Light source (124) is configured to generate infrared light (e.g., at a specific wavelength) and communicate the infrared light to optical cable (122). Various suitable forms and configurations that light source (124) may take will be apparent to those of ordinary skill in the art in view of the teachings herein. Camera (130) is positioned over the patient, with the line of sight of camera (130) being directed at the patient's face. Camera (130) is configured to detect infrared light communicated through tissue of the patient's face. Various suitable forms and configurations that camera (130) may take will be apparent to those of ordinary skill in the art in view of the teachings herein. Camera (130) is coupled with processor (126) by a cable (132). Processor (126) is operable to process video signals received from camera (130) and drive the display of corresponding video on display (140). Display (140) may comprise a monitor or any other suitable kind of display. The video image displayed on display (140) includes the patient's face and any infrared light being transmitted transcutaneously through the patient's face by guidewire (110) from within the patients sinuses, etc.

It should be understood that the effectiveness of illuminated guidewire system (100) may be less dependent on ambient conditions and patient physiology than a system where guidewire (50) just emits light within the visible spectrum. By way of example only, light within the infrared spectrum may pass through tissue more easily than light within the visible spectrum. In addition or in the alternative, the sensitivity of camera (130) to infrared light emitted through the patient's face may be less affected by ambient lighting conditions than the sensitivity of a person's naked eye to visible light emitted through the patient's face. In other words, illuminated guidewire system (100) of the present example may be operable and effective even when the room in which the patient is situated is fully lit and even if the patient's physiology would not be sufficiently compatible with a system using guidewire (50).

FIG. 7 shows a set of glasses (200) that may be used as a substitute for a separate camera (130) and display (140) in illuminated guidewire system (100). Glasses (200) may be worn by the person performing the sinuplasty procedure. Glasses (200) include a camera (230) and an eyescreen (240) that is positioned in front of the wearer's eyes. Camera (230) is substantially similar to camera (130) described above in that camera (130) is operable to detect infrared light emitted through tissue in the patient's face. Of course, the line of sight for camera (230) in this example will move with the operator's head; whereas the line of sight for camera (130) is substantially fixed. Eyescreen (240) includes an imaging overlay (242) that is operable to display video captured by camera (130), including visual representations of infrared light captured by camera (130). Eyescreen (240) is transparent. In some versions, imaging overlay (242) is translucent or semi-transparent such that overlay (242) does not completely obstruct the wearer's view. Imaging overlay (242) may occupy any suitable area of eyescreen (240) and may be located at any suitable position on eyescreen (240). Various suitable components that may be used to form overlay (242) will be apparent to those of ordinary skill in the art in view of the teachings herein. It should also be understood that overlay (242) may also be used in settings where glasses (200) lack camera (230). For instance, some versions of glasses (200) may be coupled with a separate camera (130), such that overlay (242) displays images captured by camera (130).

Glasses (200) of the present example also include a cable (232), which may be coupled with processor (126) to provide image processing, electrical power, etc. In some other versions, cable (232) is omitted. By way of example only, glasses (200) may communicate with processor (126) wirelessly. As another merely illustrative example, glasses (200) may have on-board processing, such that a separate processor (126) is unnecessary.

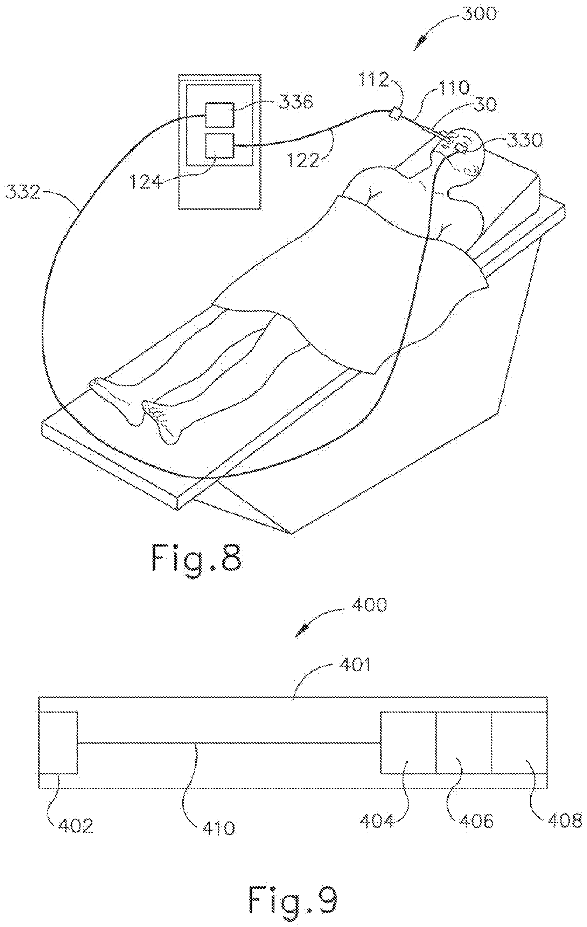

FIG. 8 shows an embodiment of an illuminated guidewire system (300) according to the claimed invention that uses infrared light. System (300) of this embodiment is a variation of system (100) above. In particular, system (300) of this embodiment comprises an illuminated guidewire (110) with a connector (112), optical cable (122), and infrared light source (124) just like the same components referred to above. However, instead of having a camera (130) and display (140), system (300) of this embodiment includes an infrared sensitive photodetector (330) placed on the patient's skin over the targeted area (e.g., on the patient's cheek, etc.). In some versions, photodetector (330) is configured as a patch that adheres to skin. Photodetector (330) is configured to register infrared light from guidewire (110) when guidewire (110) has reached the targeted area (e.g., maxillary sinus, etc.). Photodetector (330) is coupled with a feedback device (336) via a cable (332). Feedback device (336) is operable to process signals from photodetector (330) and provide the operator with feedback (e.g., audible feedback, visual feedback, etc.) indicating detection of infrared light by photodetector (330). In addition or in the alternative, photodetector (330) may include an integrated feedback feature that is configured to provide the operator with feedback (e.g., audible feedback, visual feedback, etc.) indicating detection of infrared light by photodetector (330), such that cable (332) may be omitted.

Various suitable forms that photodetector (330) may take, as well as various ways in which photodetector (330) may be implemented to sense proper placement of guidewire (110), will be apparent to those of ordinary skill in the art in view of the teachings herein. It should also be understood that photodetector (330) may apply pressure to the skin, which may increase the sensitivity of photodetector (330) by removing perfusion and reducing optical scattering by the dermis. Such pressure may be enhanced by providing complementary magnets on photodetector (330) and within the distal end of guidewire (110), such that the two are magenetically attracted to each other and thereby increase the pressure as they get closer together. Of course, the use of photodetector (330) need not be limited to infrared light contexts. The use of photodetector (330) could be readily applied to contexts where an illuminating guidewire (50) emits light in the visible spectrum.

### B. Exemplary LED Illuminated Guidewire

FIG. 9 shows another exemplary illuminated guidewire (400) that comprises a body (401) supporting a battery (402), a light source (404), a beam processing element (406), and a lens (408). The body (401) of guidewire (400) may be configured similar to the body of guidewire (50) described above; similar to any of the guidewire bodies described in U.S. Pub. No. 2012/0078118; and/or in any other suitable fashion. Guidewire (400) is sized for insertion through guide catheter (30) in a patient's nose, just like guidewire (50). In some versions, guidewire (400) has one or more embedded optical fibers that have a bend radius of approximately 5 mm or less. Some versions of guidewire (400) may also have a numerical aperture to provide more than approximately 10 W/cm² of irradiance or a total of more than approximately 4 mW of power at the distal end of guidewire (400).

Light source (404) comprises a narrowband low power (e.g., between approximately 5mW and approximately 3W) solid state light source. In some versions, light source (404) comprises a light emitting diode (LED). In some versions where light source (404) comprises an LED, the LED has an output power of more than 150 mW. In some other versions, light source (404) comprises a diode laser. In some versions where light source (404) comprises a diode laser, the diode laser has an output power of more than 5mW. Light source (404) may provide light at any suitable wavelength. By way of example only, light source (404) may provide light at a wavelength or range of wavelengths between approximately 610 nm and approximately 700 nm.

Battery (402) is coupled with light source (404) via a wire (410) and is thereby operable to provide electrical power to light source (404). Battery (402) is positioned in the proximal end of guidewire (400) in this example though it should be understood that battery (402) may instead be positioned further distally in guidewire (400). In some versions, battery (402) is directly coupled with light source (404) such that wire (410) may be omitted. It should therefore be understood that battery (402) and/or light source (404) may be located either at the proximal end of guidewire (400), near the distal end of guidewire (400), or anywhere in between. It should also be understood that light source (404) may receive electrical power from some other source. By way of example only, light source (404) may be coupled with an external power source via one or more wires extending from guidewire (400).

Beam processing element (406) is optically interposed between light source (404) and lens (408). In some versions, beam processing element (406) is secured directly to a distal portion of light source (404) or is integrated within light source (404). In some other versions, beam processing element (406) is optically coupled with light source (404) via one or more optical fibers, light pipes, etc. Similarly, it should be understood that beam processing element (406) may be secured directly to lens (408) or may be otherwise integral with lens (408). In some other versions, beam processing element (406) is optically coupled with lens (408) via one or more optical fibers, light pipes, etc. It should therefore be understood that one or both of light source (404) or beam processing element (406) may be located in the proximal end of guidewire (400) while lens (408) is located at the distal end of guidewire (400), with optical fiber providing a path for communication of light from the proximal end toward the distal end of guidewire (400).

Beam processing element (406) is operable to optically process light emitted by light source (404) before the light reaches lens (408). By way of example only, beam processing element (406) may provide a beam waist with an average irradiance of more than 10 W/cm² within the acceptance angle of the optical fiber in guidewire (400) and at incidence to the distal end of optical fiber in guidewire (400). Beam processing element (406) may comprise one or more aspheric, anamorphic, and/or graded-index (GRIN) lenses. It should also be understood that a diffuser, optical phase modulator, and/or various other optical components may be incorporated into guidewire (400). For instance, a diffuser may be located at the distal end of guidewire (400) or anywhere proximal thereto. As one merely illustrative example where light source (404) comprises a laser light source, an optical phase modulator is used to alter light source (404) to a non-coherent source. Other suitable components that may be used to form a beam processing element (406) will be apparent to those of ordinary skill in the art in view of the teachings herein.

It should be understood from the foregoing that guidewire (400) may provide a significant improvement in electrical-to-optical efficiency over a convention illuminated guidewire (50). Guidewire (400) may also operate at significantly lower temperatures, thereby providing a reduced risk of thermal damage to optical fiber, etc. Some operators (400) may also appreciate the portability of guidewire (400) since the power source (battery (402)) is incorporated directly into guidewire (400). Furthermore, providing a relatively narrowband light source (404) may result in a more efficient use of energy for tissue penetration.

For versions of guidewire (400) that are tuned to provide light at a specific wavelength (e.g., where an LED serves as light source (404)), the operator may wear bandpass filtering glasses (or a bandpass filtering loupe, monocle, or visor, etc.) that effectively dim ambient light but allow the specific wavelength to pass through. As with the infrared versions of guidewire (110), this kind of system may promote visualization of light from guidewire (400) even when the ambient lighting of the room is relatively bright. Various suitable films or other types of filters that may be incorporated into glasses, etc., to provide the bandpass filtering of light will be apparent to those of ordinary skill in the art in view of the teachings herein.

### IV. Miscellaneous

It should be understood that any of the examples described herein may include various other features in addition to or in lieu of those described above. By way of example only, any of the examples described herein may also include one or more of the various features disclosed in any of the various references.

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

Versions described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by a user immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometries, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. An apparatus (300) comprising:
(a) a guidewire body (110), wherein the guidewire body (110) includes an optically transmissive element, wherein the guidewire body (110) is sized for insertion in a sinus ostium of a patient;
(b) a light source (124) in optical communication with the optically transmissive element (122), wherein the light source (124) is operable to transmit infrared light through the optically transmissive element; and
(c) a detector (330) configured to detect infrared light transmitted by the optically transmissive element through the patient wherein the detector (330) comprises an infrared sensitive photodetector (330) configured as a patch to adhere to skin.

2. The apparatus (300) of claim 1, wherein the photodetector (330) further comprises a magnet configured to attract to a feature of the guidewire body (110).

3. The apparatus (300) of claim 1 or claim 2, further comprising a feedback device (336) coupled with the photodetector (330), wherein the feedback device (336) is configured to provide an operator with feedback relating to infrared light detected by the photodetector (330).

4. The apparatus (300) of claim 3 wherein the feedback device (336) is coupled with the photodetector (330) via a cable (332).

5. The apparatus (300) of claim 1 or claim 2, wherein the photodetector (330) includes an integrated feedback feature that is configured to provide the operator with feedback indicating detection of infrared light by photodetector (330).

6. The apparatus (300) of any one of claims 3 - 5 wherein the feedback is audible feedback or visual feedback.

7. The apparatus (300) of any preceding claim, wherein the light source (124) is remote from the guidewire body (110), wherein the light source (124) and the guidewire body (110) are coupled via an optical cable (122).

## Patentansprüche

1. Vorrichtung (300), umfassend:
(a) einen Führungsdrahtkörper (110), wobei der Führungsdrahtkörper (110) ein optisches Transmissionselement beinhaltet, wobei der Führungsdrahtkörper (110) zur Einführung in ein Nasennebenhöhlen-Ostium eines Patienten bemessen ist;
(b) eine Lichtquelle (124) in optischer Kommunikation mit dem optischen Transmissionselement (122), wobei die Lichtquelle (124) funktionsfähig ist, Infrarotlicht durch das optische Transmissionselement zu transmittieren; und
(c) einen Detektor (330), der dazu ausgelegt ist, Infrarotlicht zu detektieren, das von dem optischen Transmissionselement durch den Patienten transmittiert wird, wobei der Detektor (330) einen infrarotempfindlichen Photodetektor (330) umfasst, der als ein auf der Haut haftendes Pflaster konfiguriert ist.

2. Vorrichtung (300) nach Anspruch 1, wobei der Photodetektor (330) ferner einen Magneten umfasst, der dazu ausgelegt ist, zu einem Merkmal des Führungsdrahtkörpers (110) angezogen zu werden.

3. Vorrichtung (300) nach Anspruch 1 oder Anspruch 2, ferner umfassend eine Rückmeldeeinrichtung (336), die mit dem Photodetektor (330) gekoppelt ist, wobei die Rückmeldeeinrichtung (336) dazu ausgelegt ist, einem Bediener eine Rückmeldung bezüglich Infrarotlicht, das durch den Photodetektor (330) detektiert wird, bereitzustellen.

4. Vorrichtung (300) nach Anspruch 3, wobei die Rückmeldeeinrichtung (336) über ein Kabel (332) mit dem Photodetektor (330) gekoppelt ist.

5. Vorrichtung (300) nach Anspruch 1 oder Anspruch 2, wobei der Photodetektor (330) ein integriertes Rückmeldemerkmal beinhaltet, das dazu ausgelegt ist, dem Bediener eine Rückmeldung bereitzustellen, die eine Detektion von Infrarotlicht durch den Photodetektor (330) angibt.

6. Vorrichtung (300) nach einem der Ansprüche 3-5, wobei die Rückmeldung eine hörbare Rückmeldung oder eine visuelle Rückmeldung ist.

7. Vorrichtung (300) nach einem vorangegangenen Anspruch, wobei sich die Lichtquelle (124) entfernt von dem Führungsdrahtkörper (110) befindet, wobei die Lichtquelle (124) und der Führungsdrahtkörper (110) über ein optisches Kabel (122) gekoppelt sind.

## Revendications

1. Appareil (300) comprenant :
(a) un corps de fil guide (110), le corps de fil guide (110) comportant un élément optiquement transmissif, le corps de fil guide (110) étant dimensionné pour insertion dans un ostium sinusien d'un patient ;
(b) une source de lumière (124) en communication optique avec l'élément optiquement transmissif (122), la source de lumière (124) étant utilisable pour transmettre de la lumière infrarouge à travers l'élément optiquement transmissif ; et
(c) un détecteur (330) configuré pour détecter la lumière infrarouge transmise par l'élément optiquement transmissif à travers le patient
dans lequel le détecteur (330) comprend un photodétecteur sensible aux infrarouges (330) configuré comme un timbre pour coller à la peau.

2. Appareil (300) de la revendication 1, dans lequel le photodétecteur (330) comprend en outre un aimant configuré pour attirer un élément du corps de fil guide (110) .

3. Appareil (300) de la revendication 1 ou la revendication 2, comprenant en outre un dispositif de retour (336) couplé au photodétecteur (330), le dispositif de retour (336) étant configuré pour fournir à un opérateur un retour concernant la lumière infrarouge détectée par le photodétecteur (330).

4. Appareil (300) de la revendication 3 dans lequel le dispositif de retour (336) est couplé au photodétecteur (330) par un câble (332).

5. Appareil (300) de la revendication 1 ou la revendication 2, dans lequel le photodétecteur (330) comporte un élément de retour intégré qui est configuré pour fournir à l'opérateur un retour indiquant la détection de lumière infrarouge par le photodétecteur (330) .

6. Appareil (300) de l'une quelconque des revendications 3 à 5 dans lequel le retour est un retour audible ou un retour visuel.

7. Appareil (300) d'une quelconque revendication précédente, dans lequel la source de lumière (124) est distante du corps de fil guide (110), la source de lumière (124) et le corps de fil guide (110) étant couplés par un câble optique (122).
